# EUROPEAN PATENT SPECIFICATION

(11) **EP 0 776 224 B1**
(45) Date of publication and mention of the grant of the patent: **09.12.1998**
(21) Application number: 95928575.0
(22) Date of filing: 17.08.1995
(51) Int. Cl.: A61M 5/30, C12M 3/00

(54) **NEEDLELESS SYRINGE FOR PARTICLE DELIVERY**
NADELLOSE SPRITZE FÜR DIE ABGABE FEINER TEILCHEN
SERINGUE SANS AIGUILLE POUR ADMINISTRER DES PARTICULES

(30) Priority: 17.08.1994 GB 9416663
(43) Date of publication of application: 04.06.1997
(73) Proprietor: PowderJect Research Limited, Oxford OX4 4GA (GB)
(72) Inventor: BELLHOUSE, Brian, John, Islip, Oxfordshire OX5 2SQ (GB); DRAYSON, Paul, Rudd, Oxfordshire OX8 5RN (GB); GREENFORD, John, Christopher, Oxfordshire OX14 1DF (GB); POTTER, Charles, David, Ogilvy, Cambridge CB4 1NP (GB); SARPHIE, David, Francis, Oxfordshire OX8 5SH (GB)
(74) Representative: Jackson, Peter Arthur
(86) International application number: PCT/GB95/01948
(87) International publication number: WO 96/04947

(56) References cited:
- WO-A-91/00915
- DE-B- 1 100 883
- GB-A- 1 049 780

## Description

In our earlier international patent application No. WO 94/24263, which is only relevant under Article 54 (3) EPC, we disclose a non-invasive drug delivery system involving the use of a needleless syringe which fires light drug-containing particles in controlled doses into the intact skin or delivers genetic material into living cells. The syringe described in the earlier application is constructed as an elongate tubular nozzle, a rupturable membrane initially closing the passage through the nozzle adjacent to the upstream end of the nozzle, particles of a therapeutic agent, particularly a powdered therapeutic agent, located adjacent to the membrane, and energizing means for applying to the upstream side of the membrane a gaseous pressure sufficient to burst the membrane and produce through the nozzle a supersonic gas flow in which the particles are entrained.

By appropriate selection of the geometry and Mach number for the nozzle, which preferably has a convergent upstream portion, leading through a throat to a cylindrical or, preferably, divergent downstream portion, it has been possible to provide a pseudo-steady state, supersonic two phase flow through the nozzle, in which the particles move with a velocity close to that of the propelling gas in which they are entrained. Consequently, a large proportion of the particles reach the target under quasi-steady flow conditions and only a small proportion are delivered in transient flow and carried on the contact surface. This leads to considerable benefit both in control and in increased skin or other target penetration and is surprising in such a transient phenomenon. High speed photography of the gas/drug jet has confirmed the quasi-steady flow conditions. Typical photographs of the jet show the jet lasting for 1.5 milliseconds, with reasonably homogenous distribution of the drug particles throughout the jet. The length of time that the jet lasts allows us to calculate the effective length of the jet and hence its volume. This allows us to conclude that the drug particles are arriving in a continuous flow at the skin surface and that on average succeeding particles will penetrate in the same holes as preceding particles, reducing damage and trauma to the skin. This understanding has led to our appreciation that the drug dose may be advantageously mixed with the driving gas in the gas canister. The drug delivery system can then be considerably simplified as a rupturable membrane may, in most cases, no longer be needed. Now the outlet from the chamber may lead directly to the nozzle via a valve or other means for releasing the gas. This differentiates our technique from other prior art, such as EPA-0535005, which relies upon the impact of a shock wave to accelerate particles, whereas our technique accelerates them in a flow of gas.

WO 91/00915 discloses a needleless syringe constructed as an elongate nozzle at the upstream end of which is provided a sealed chamber containing gas at superatmospheric pressure, and means for opening an outlet from the chamber to release the gas whereby gas and particles of an agent entrained with the gas flow through the nozzle at supersonic speed. In order to mix the particles of the agent with the gas a nebuliser is arranged downstream of the gas chamber and upstream of the nozzle.

In accordance with the invention, however, the needleless syringe is characterised in that the sealed chamber contains gas at super-atmospheric pressure and particles of a therapeutic agent. The location of the particles within the chamber which contains the high pressure gas considerably simplifies the construction and assembly of the syringe.

The outlet from the chamber may incorporate a pierceable membrane or a valve, such as a spring-loaded ball valve, which is pierced or opened by manual manipulation, such as by movement of two parts of the syringe relatively to one another.

The chamber may contain a single dose of particles, and hence sufficient gas for a single shot. Alternatively, the outlet from the chamber may incorporate a valve which may be opened and closed a consecutive number of times to deliver a succession of doses of the therapeutic agent. The time during which the valve is opened may be automatically controlled by control means, for example including a solenoid or stepping motor the valve being opened for successively longer periods to deliver equal doses of therapeutic agent in spite of the successively reducing pressure in the chamber. It will then be appreciated that by placing the drug in the gas canister, creating a homogeneous mixing and suspension of the powdered drug and by using a fast opening metering valve at the exit of the canister, a multi-shot syringe may be created, dispensing an infinitely variable, rather than a unit, dose of drug. By combining the metering valve with a timing device, the duration of time that the valve is open for each shot may be controlled. The timer may be adjusted to take account of the desired dose of drug, the gas pressure in the reservoir and the drug concentration in the reservoir (both of which will decrease with each shot). If the initial values of the drug mass and the gas pressure in the reservoir are known, then the required duration of each subsequent shot can be calculated, eg by a microprocessor, and the timer adjusted accordingly.

Alternatively, when the actual dose is not critical, for example when the therapeutic agent is an analgesic, the valve may be opened and closed manually. A degree of control may then be provided by means of a rupturable membrane between the valve and the nozzle, the valve being opened to release sufficient gas and particles into a rupture chamber upstream of the membrane until the pressure across the membrane has built up sufficiently for the membrane to rupture, whereafter the particles, entrained in the gas, are free to flow from the rupture chamber through the nozzle to the target. As soon as the operator hears the membrane burst, he will reclose the valve, for example by releasing a trigger which holds the valve open against the spring pressure.

The sealed chamber containing the gas under pressure and the particles may be supplied as a separate unit to be united, at the time of use, with another part of the syringe incorporating the tubular nozzle, and possibly also a diaphragm-piercing or valve-opening device.

One slight disadvantage with the earlier syringe, in which gas pressure was used to burst the rupturable membrane, was the difficulty in ensuring that the full prescribed dose of particles was delivered in the gas flow, without any remaining in the proximity of the remnants of the burst membrane. With the new syringe, this disadvantage can be overcome if the particles are initially located in one or more open ended passageways within the chamber, and arranged such that upon opening of the outlet and release of the compressed gas, at least some of this gas sweeps through the passageway(s) and thus entrains substantially all the particles within the passageway(s). The particles may be initially retained in the passageways under gravity, or electrostatically, or by means of weak membranes closing the ends of the passageways until ruptured by the release of pressure. Another solution involves making the particles of drug to be so small as to remain suspended in the gas for a few seconds when agitated but big enough so as to not enter the skin cells and reduce bio-availability. On the contrary, the drug particles occupy extra-cellular space and hence diffuse readily into the systemic circulation. Particle diameters of between 10-20µm are preferable. A ball bearing may be placed inside the gas canister to help homogenous distribution of the drug upon shaking, prior to firing.

Study of the drug jet and its arrival at the target surface has further led to the appreciation that the jet dimensions are important and affect the concentration of drug per unit volume of target skin. By increasing the volume of driver gas one may lengthen the duration of the jet and increase the dosage of drug that may be delivered, subject to the limitation of drug concentration that is viable in the skin. For a given concentration, dosage delivered may also be increased by increasing target skin area. This may be achieved by increasing the diameter of the throat of the nozzle, whilst maintaining constant the ratio of inlet and exit diameters. Target diameter may also be increased by increasing the spacing distance of the nozzle exit from the target.

At the downstream end of the nozzle a spacer/silencer may be used. The type of particle which may be delivered, can be a powdered therapeutic agent.

Some examples of syringes constructed in accordance with the present invention are illustrated diagrammatically in the accompanying drawings, in which:
Figure 1 is an axial section through a first example;
Figure 2 is a side elevation of the first example;
Figures 3 and 4 correspond to Figures 1 and 2 but show a second example;
Figures 5 and 6 show, to an enlarged scale, parts of Figures 3 and 4;
Figures 7 and 8 correspond to Figures 5 and 6 but show a third example;
Figures 9 and 10 correspond to Figures 1 and 2 but show a fourth example;
Figure 11 is a diagrammatic axial view of a canister;
Figure 12 is a section taken on the XII-XII in Figure 11;
Figure 13 is an exploded view of another example;
Figure 14 is an enlargement of part of Figure 13; and,
Figure 15 is a view of a further example.

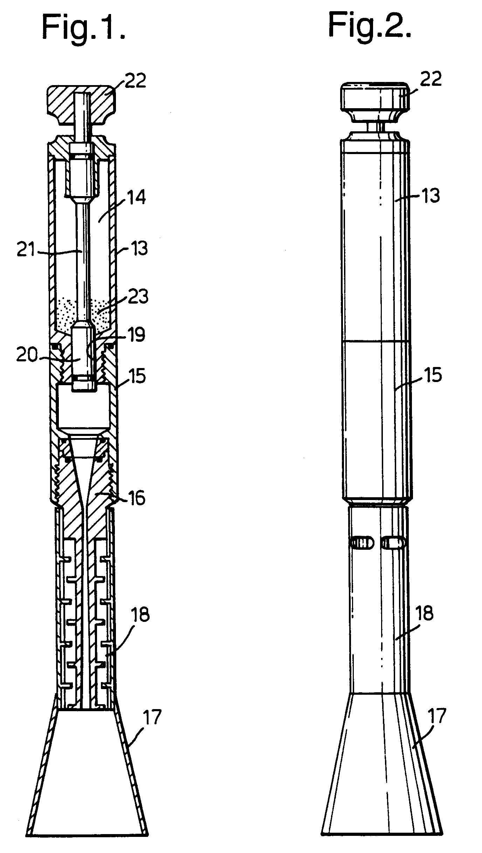

The syringe shown in Figures 1 and 2 is almost identical to the syringe shown in Figures 1 to 3 of the earlier application WO 94/24263, in having an upper barrel portion 13 containing a sealed reservoir or chamber 14 and coupled by screw threads via a lower barrel portion 15 to a nozzle 16 associated with a spacer 17 and silencer 18. An outlet 19 in the bottom of the chamber 14 is closed by a valve element 20 on the end of a plunger 21 which is depressible by a button 22. The syringe differs from that in the earlier application in not having at the top, upstream end of the nozzle 16 a capsule consisting of two rupturable membranes between which the particles of therapeutic agent are isolated. Instead, these particles 23 are located within the chamber 14. When the particles are to be delivered, the button 22 is depressed thereby moving the sealed carrying part of the element 20 downwardly out of the outlet 19, and releasing the compressed gas and particles in the chamber 14 to flow at supersonic speed through the nozzle 16 to a target positioned beyond the spacer 17.

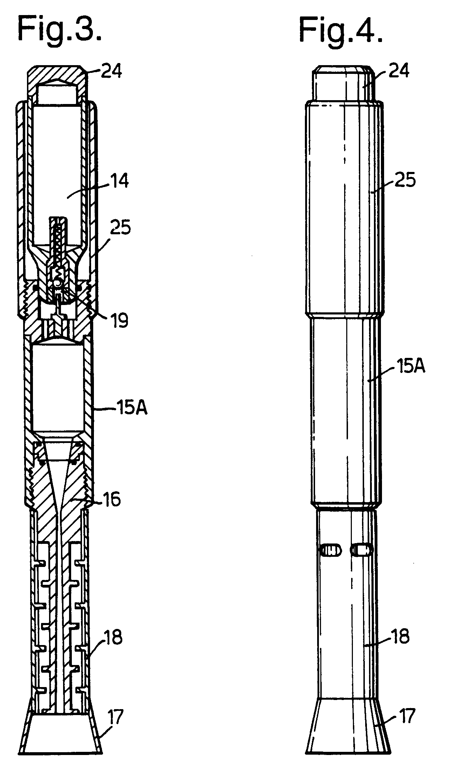

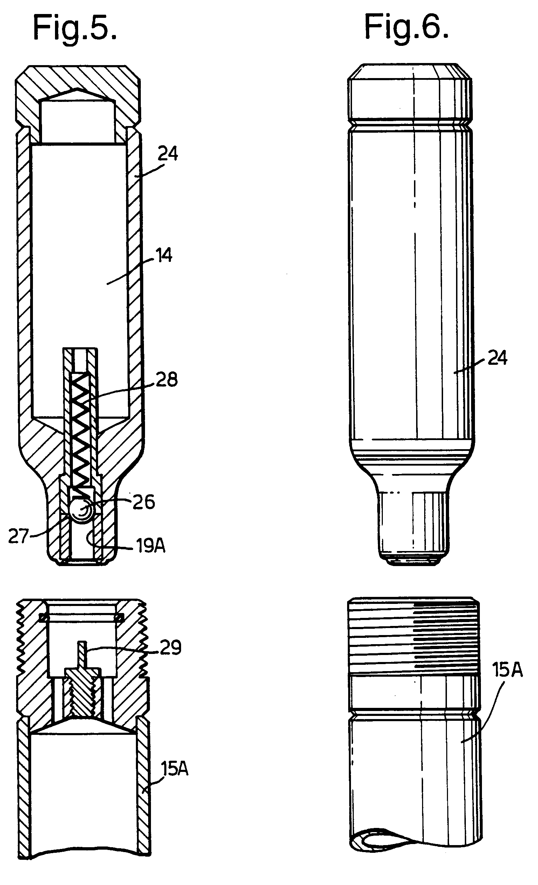

The syringe shown in Figures 3 to 6 differs from that shown in Figures 1 and 2 in that the chamber 14 is provided within a canister 24 which is slidable within a sleeve 25 corresponding to the upper barrel portion 13 in the first example. An outlet 19A is closed by a valve consisting of a ball closure element 26 which is urged onto a seating 27 by a helically coiled compression spring 28. Within the lower barrel portion 15A there is provided an upwardly projecting spigot 29 which can enter the outlet 19A and, when the upper end of the canister 24 is pressed downwardly into the upper barrel portion 25, displaces the ball closure element 26 from its seat and allows sudden release of the gas and particles which are contained within the chamber 14. The valve may be reclosed, for multi shot use by releasing the pressure on the canister.

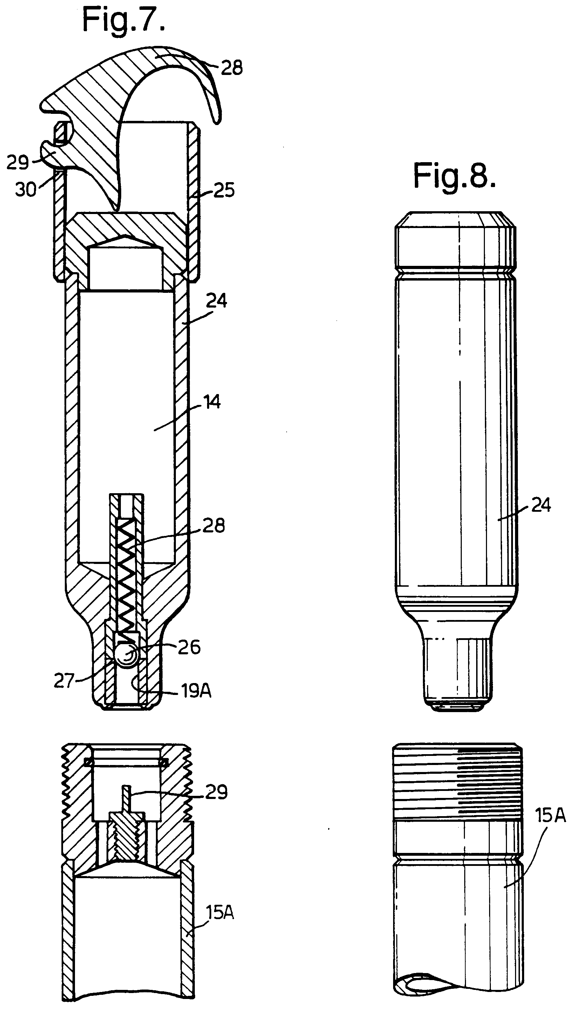

The modification shown in Figures 7 and 8 differs from that of the Figures 3 to 6 example only in that a thumb piece 28 is pivotally mounted in the top of the barrel portion 25 by means of a lug on the thumb piece engaging an aperture 30 in the barrel portion. Depression of the thumb piece forces the bulb 24 downwards within the barrel portion 25 and provides a mechanical advantage which facilitates movement of the ball closure element against the high pressure within the chamber 14.

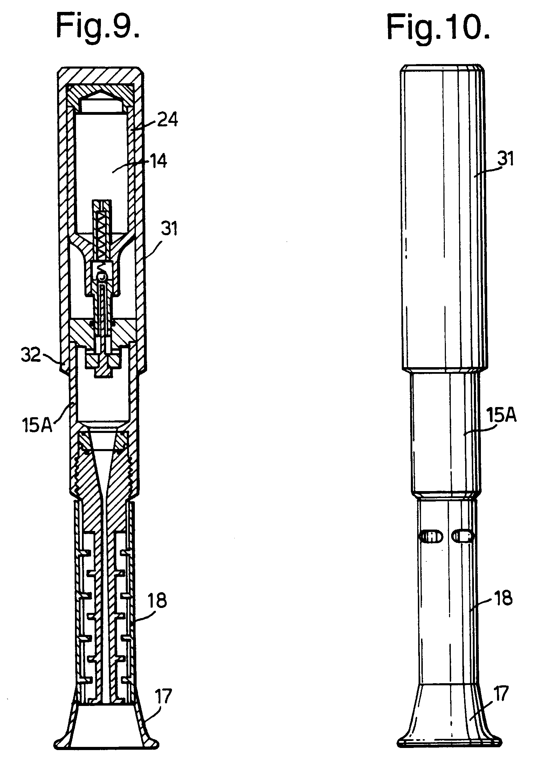

Figures 9 and 10 show an alternative way of forcing the canister 24 downwardly to open the ball valve. In this example a cylindrical shroud 31 is slipped down over the canister 24 and its lower end 32 slides on the lower barrel portion 15A. Downward pressure on the closed upper end of the shroud 31 forces the canister 24 downwards to open the valve.

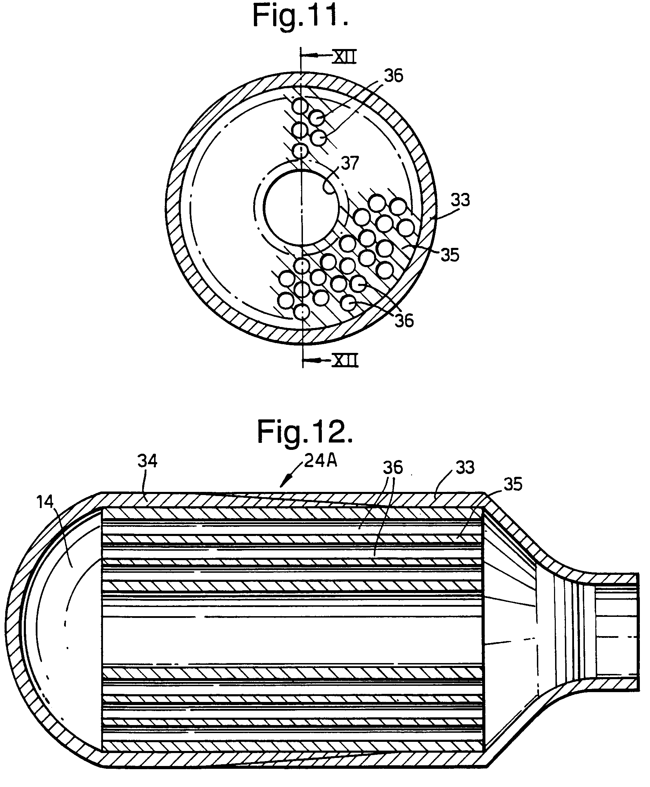

The pressure containing parts of the syringe will usually be made of metal, but may also be made of a rigid engineering plastics material, such as a polycarbonate. A canister 24A made of such plastics material is shown in Figures 11 and 12. The body of the canister is made in two parts 33 and 34 which are fused or welded together. The canister contains an insert 35 consisting of a number of parallel cylindrical passageways 36. Upon assembly, the canister is filled with high pressure gas and the particles of therapeutic agent are located within the passageways 36. An outlet 37 of the canister may be fitted with a ball valve such as in the previous examples, or closed by means of a pierceable diaphragm which is sufficiently strong to contain the internal gas pressure but which may be readily breached by a needle, which may be hollow, when the canister is moved relatively to the needle.

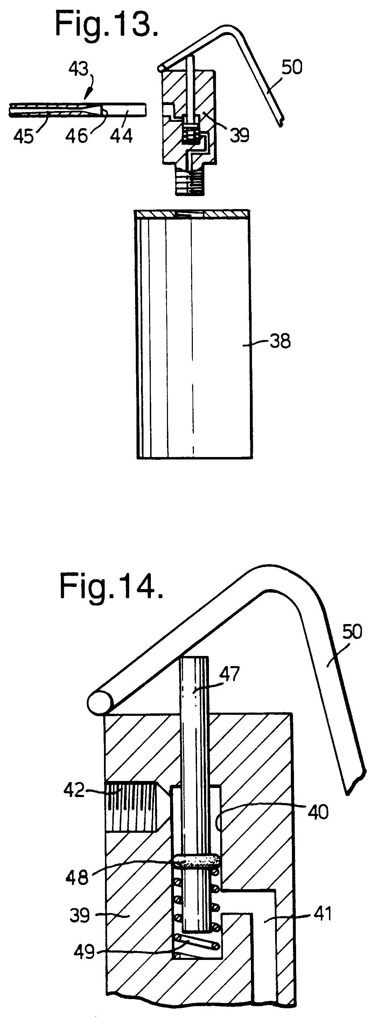

Figures 13 and 14 show a multi dose syringe which comprises a canister 38 containing compressed gas and particles of therapeutic agent, together with an agitator, such as a metal ball. Prior to discharge, the canister is shaken so that the particles are suspended in the gas and entrained by the gas when it is released.

Screwed into the lid of the canister is a valve housing 39 containing a valve chamber having a lower side passage 41 leading down into the canister 38 into which it opens, and an upper side passage 42 into which is screwed a nozzle assembly 43 consisting of a rupture chamber 44 and a nozzle 45 separated by a rupturable membrane 46. A plunger 47 extends into the valve chamber 40 and carries an O sealing ring 48. The plunger is urged upwardly by a helically coil compression spring 49 so that the ring 48 is above the side passage 41. When the plunger 47 is depressed against the action of the spring, by downward displacement of an L-shaped lever 50, which is pivoted to the valve housing 39, the ring 48 rides to below the passage 41, allowing the escape of a suspension of the particles in the conveying gas from the canister 38 and into the rupture chamber 44. When the pressure in the chamber 44 has built up sufficiently, the membrane 46 bursts and the particles, entrained in a supersonic gas flow, are ejected through the nozzle 45. The lever 50 is conveniently manipulated by grasping the canister 38 in the palm of the user's hand, and depressing the free end of the lever with the thumb. As soon as the user hears the membrane burst, the lever 50 can be released, so that the plunger 47 is raised under the action of the spring 49, effectively reclosing the valve ready for a subsequent shot, prior to which the membrane 46 will need to be replaced, for example by unscrewing the nozzle 45 from the part of the assembly 43 containing the rupture chamber 44.

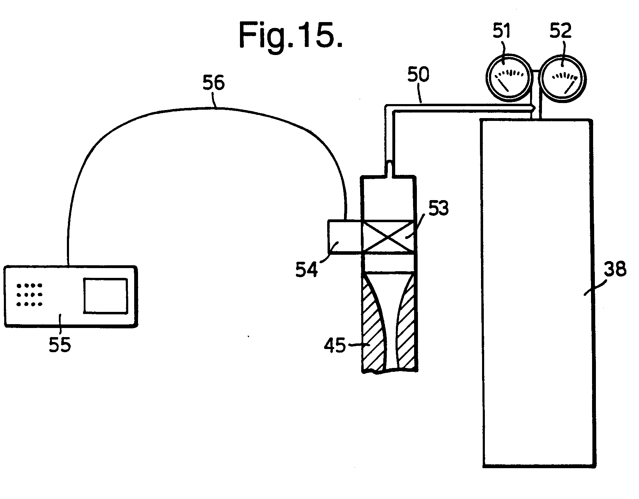

The system shown in Figure 15 differs from that of Figure 13 and 14 in that the canister 38 of gas and particles is connected to the nozzle 45 via tubing 50, which may be rigid or flexible, containing a pressure regulator 51 and a pressure indicator 52, and a valve 53. The valve is a fast acting valve which is controlled by a solenoid or stepping motor 54 under the control of a micro processor 55, which is connected to the control 54 by an umbilical 56. In this example the valve 53 is opened according to the timing programme of the micro processor 55, for a predetermined time to cause the required dose to be ejected through the nozzle 45. The micro processor will be programmed not only with the required dose but with the initial pressure in the canister 38 and the concentration of particles of therapeutic agent, so that the required dose can be repeated irrespective of reducing pressure in the canister 38.

Typically, in each of the illustrated examples, the gas provided in the chamber 14 may be helium at a pressure of the order of 40 to 80 bar. The nozzle may be of convergent/divergent, or convergent/cylindrical form with a length of between 50 and 100, preferably 60mm, and a throat diameter of between 1 and 10, preferably between 1.5 and 5mm. With appropriate gas pressure, particles having a diameter of 10-40µm will be accelerated through the nozzle to velocities of between Mach 1 and 3.

## Claims

1. A needleless syringe comprising an elongate nozzle (16, 45) to the upstream end of which is connected a sealed chamber (14, 38) containing gas at super-atmospheric pressure, and means (20, 26, 47, 53) for opening an outlet (19, 19A, 37) from the chamber to release the gas whereby gas and particles of an agent entrained with the gas flow through the nozzle at supersonic speed; characterised in that the sealed chamber (14, 38) contains gas at super-atmospheric pressure and particles (23) of a therapeutic agent.

2. A syringe according to claim 1, in which the outlet from the chamber (14, 38) incorporates a pierceable membrane or a valve (20; 26,27; 40,48; 53) which is arranged to be pierced or opened, respectively, by manual manipulation.

3. A syringe according to claim 2, in which the manual manipulation involves movement of two parts (13,21; 24,15A; 39,50) of the syringe relatively to one another.

4. A syringe according to claim 2, in which the outlet from the chamber (38) incorporates a valve (53) which may be opened and closed a consecutive number of times, to deliver a succession of doses of the therapeutic agent.

5. A syringe according to claim 4, in which the time during which the valve (53) is opened is automatically controlled by control means (55), and the valve (53) is opened for successively longer periods to deliver equal and selected doses of therapeutic agent in spite of the successively reducing pressure in the chamber.

6. A syringe according to claim 4 or claim 5, in which the control means (55) includes a solenoid or stepping motor (54).

7. A syringe according to any one of the preceding claims, in which the sealed chamber contains one or more open ended passageways (36) and arranged such that, upon opening of the outlet, and release of the compressed gas, at least some of this gas sweeps through the passageway(s) (36) and entrains the particles within the passageway (36).

8. A syringe according to any one of the preceding claims, wherein the particles (23) are of a powdered therapeutic agent.

## Patentansprüche

1. Nadellose Spritze, enthaltend eine längliche Düse (16, 45), mit deren stromaufwärts gelegenem Ende eine abgedichtete Kammer (14, 38) verbunden ist, die Gas mit einem Druck über dem atmosphärischen Druck enthält, und eine Einrichtung (20, 26, 47, 53) zum Öffnen eines Auslasses (19, 19A, 37) aus der Kammer, um das Gas freizugeben, so daß Gas und Partikel eines Mittels, die mit dem Gas mitgerissen werden, mit Überschallgeschwindigkeit durch die Düse fließen; dadurch gekennzeichnet, daß die abgedichtete Kammer (14, 38) Gas mit einem Druck über dem atmosphärischen Druck und Partikel (23) eines therapeutischen Mittels enthält.

2. Spritze nach Anspruch 1, bei welcher der Auslaß aus der Kammer (14, 38) eine durchstechbare Membran oder ein Ventil (20; 26, 27; 40, 48; 53) enthält, die bzw. das so ausgelegt ist, daß sie bzw. es durch Betätigung von Hand durchstochen bzw. geöffnet werden können.

3. Spritze nach Anspruch 2, bei welcher die Betätigung von Hand die Bewegung von zwei Teilen (13, 21; 24, 15A; 39, 50) der Spritze relativ zueinander einschließt.

4. Spritze nach Anspruch 2, bei welcher der Auslaß aus der Kammer (38) ein Ventil (53) enthält, das mehrmals aufeinanderfolgend geöffnet und geschlossen werden kann, um eine Abfolge von Dosen des therapeutischen Mittels abzugeben.

5. Spritze nach Anspruch 4, bei welcher die Zeit, während welcher das Ventil (53) geöffnet ist, durch eine Steuereinrichtung (55) automatisch gesteuert wird, und das Ventil (53) für sukzessive längere Zeiträume geöffnet wird, um trotz des sukzessive abnehmenden Druckes in der Kammer gleiche und ausgewählte Dosen des therapeutischen Mittels abzugeben.

6. Spritze nach Anspruch 4 oder Anspruch 5, bei welcher die Steuereinrichtung (55) ein Solenoid oder einen Schrittmotor (54) enthält.

7. Spritze nach einem der vorstehenden Ansprüche, bei welcher die abgedichtete Kammer einen oder mehrere Kanäle (36) mit offenem Ende enthält, die so angeordnet sind, daß beim Öffnen des Auslasses und Freigeben des komprimierten Gases mindestens ein Teil dieses Gases durch den oder die Kanäle (36) fließt und die Partikel in dem Kanal (36) mitreißt.

8. Spritze nach einem der vorstehenden Ansprüche, bei welcher die Partikel (23) aus einem pulverisierten therapeutischen Mittel bestehen.

## Revendications

1. Seringue sans aiguille, comprenant une buse allongée (16, 45) à l'extrémité amont de laquelle est raccordée une chambre étanche (14, 38) qui contient un gaz à une pression supérieure à la pression atmosphérique, et un dispositif (20, 26, 47, 53) destiné à ouvrir une sortie (19, 19A, 37) de la chambre pour libérer le gaz afin que le gaz et les particules d'un agent entraîné avec le gaz s'écoulent dans la buse à une vitesse supersonique, caractérisée en ce que la chambre étanche (14, 38) contient un gaz à une pression supérieure à la pression atmosphérique et des particules (23) d'un agent thérapeutique.

2. Seringue selon la revendication 1, dans laquelle la sortie de la chambre (14, 38) comporte une membrane perçable ou une soupape (20 ; 26, 27 ; 46, 48 ; 53) qui est destinée à être percée ou ouverte respectivement par manipulation manuelle.

3. Seringue selon la revendication 2, dans laquelle la manipulation manuelle comprend le déplacement de deux parties (13, 21 24, 15A ; 39, 50) de la seringue l'une par rapport à l'autre.

4. Seringue selon la revendication 2, dans laquelle la sortie de la chambre (38) comporte une soupape (53) qui peut être ouverte et fermée un certain nombre de fois consécutives pour la distribution d'une série de doses de l'agent thérapeutique.

5. Seringue selon la revendication 4, dans laquelle le temps pendant lequel la seringue (53) est ouverte est réglé automatiquement par un dispositif de commande (55), et la soupape (53) est ouverte pendant des périodes de plus en plus longues pour la distribution de doses d'agent thérapeutique choisies et égales malgré la diminution successive de la pression dans la chambre.

6. Seringue selon la revendication 4 ou 5, dans laquelle le dispositif de commande (55) comporte un électroaimant ou un moteur pas à pas (54).

7. Seringue selon l'une quelconque des revendications précédentes, dans laquelle la chambre étanche contient un ou plusieurs passages a extrémité ouverte (36) disposés afin que, lors de l'ouverture de la sortie et de la libération du gaz comprimé, une partie au moins du gaz balaye les passages (36) et entraîne les particules qui se trouvent dans les passages (36).

8. Seringue selon l'une quelconque des revendications précédentes, dans laquelle les particules (23) sont formées d'un agent thérapeutique en poudre.
